# EUROPEAN PATENT APPLICATION

(11) **EP 4 477 643 A1**
(43) Date of publication of application: **18.12.2024**
(21) Application number: 23749883.7
(22) Date of filing: 06.02.2023
(51) Int. Cl.: C07C 51/42, B01D 61/02, B01D 71/56, C07C 51/43, C07C 63/26, C07C 63/28, C12N 15/55, C12P 7/44

(54) **METHOD FOR PRODUCING TEREPHTHALIC ACID OR SALT THEREOF**

(30) Priority: 07.02.2022 JP 2022016865
(71) Applicant: Toray Industries, Inc., Tokyo 103-8666 (JP)
(72) Inventor: KASAHARA, Takuya, Otsu-shi, Shiga 520-8558 (JP); TSURUYA, Atsuki, Kamakura-shi, Kanagawa 248-8555 (JP); KURIHARA, Hiroyuki, Kamakura-shi, Kanagawa 248-8555 (JP)
(74) Representative: Kador & Partner Part mbB
(86) International application number: PCT/JP2023/003694
(87) International publication number: WO 2023/149567

(57) **Abstract**

An aqueous solution comprising terephthalic acid or a salt thereof and a diol is filtered across a polyamide nanofiltration membrane, and terephthalic acid or a salt thereof can be selectively separated and recovered from the non-permeated solution side and the diol can be selectively separated and recovered from the permeated solution side. The selectively separated and recovered terephthalic acid or salt thereof or diol can each be independently reutilized as a polymer starting material, e.g., for a polyester.

## Description

### TECHNICAL FIELD

The present invention relates to a method for producing terephthalic acid or a salt thereof including a step of recovering terephthalic acid or a salt thereof from a hydrolysate or the like of a terephthalic acid-based polyester such as polyethylene terephthalate (PET).

### BACKGROUND ART

In order to implement a recycling-oriented society, a chemical recycle in which plastics are depolymerized down to monomers and then the monomers are repolymerized is becoming increasingly important. Polyethylene terephthalate (PET) is also not an exception, and a methanolysis method, a glycolysis method, and the like are known. In recent years, an enzymatic chemical recycle using a PET degrading enzyme, which is one type of cutinase, has been proposed (Patent Literature 1). In the enzymatic chemical recycle of PET, it has been reported that the required energy amount and greenhouse gas emission amount can be reduced compared to the supply chain of terephthalic acid produced without recycling (Non-Patent Literature 1). On the other hand, in the chemical recycle of PET, since an aqueous solution containing the terephthalic acid and the ethylene glycol as monomer components is obtained, efficient separation and purification of each monomer component from the aqueous solution has been a challenge.

Membrane purification has been proposed as a method for separating and purifying the hydrolysate of PET, although the membrane purification has not yet been fully investigated. Specifically, it has been disclosed that by filtrating an alkaline waste liquid of PET fibers through an alkali-resistant polysulfone-based nanofiltration membrane (molecular weight cutoff 200), not only terephthalate but also ethylene glycol can be blocked to some extent on a non-permeated liquid side (Non-Patent Literature 2).

### CITATION LIST

### PATENT LITERATURE

Patent Literature 1: JP2016-505650A

### NON-PATENT LITERATURE

Non-Patent Literature 1: A.Singh et al."Techno-economic, life-cycle, and socioeconomic impact analysis of enzymatic recycling of poly(-ethylene terephthalate)" Joule,5,2021
Non-Patent Literature 2: E.K.Choe et al. "NF process for the recovery of caustic soda and concentration of disodium terephthalate from alkaline wastewater from polyester fabrics" Desalination, 186,2005

### SUMMARY OF INVENTION

### TECHNICAL PROBLEM

In a chemical recycle of terephthalic acid-based polyester, it is desirable to selectively separate and recover the terephthalic acid or a salt thereof and diol derived from terephthalic acid-based polyester, but a problem with membrane purification is that such selective separation and recovery is not possible. Therefore, an object of the present invention is to establish a membrane purification process in which terephthalic acid or a salt thereof and diol are selectively separated and recovered from an aqueous solution containing the terephthalic acid or the salt thereof and the diol.

### SOLUTION TO PROBLEM

As a result of extensive studies, the inventors have discovered that a polyamide-based nanofiltration membrane can be used as a separation membrane in a membrane purification process in which terephthalic acid or a salt thereof and diol are selectively separated and recovered, and have completed the present invention.

That is, the present invention includes the following (1) to (17).
(1) A method for producing terephthalic acid or a salt thereof, the method including a step (A) of filtrating an aqueous solution including terephthalic acid or a salt thereof and a diol through a polyamide-based nanofiltration membrane to recover the terephthalic acid or the salt thereof from a non-permeated liquid.
(2) The method for producing terephthalic acid or a salt thereof according to (1), the method further including a step (B) of hydrolyzing a terephthalic acid-based polyester to produce the aqueous solution including the terephthalic acid or the salt thereof and the diol.
(3) The method for producing terephthalic acid or a salt thereof according to (1) or (2), in which the polyamide-based nanofiltration membrane has a molecular weight cutoff of 150 to 500.
(4) The method for producing terephthalic acid or a salt thereof according to any one of (1) to (3), in which the step (A) is a step of filtrating the aqueous solution including the terephthalic acid or the salt thereof and the diol through the polyamide-based nanofiltration membrane while maintaining and/or controlling a pH at 7.0 or more and less than 11.
(5) The method for producing terephthalic acid or a salt thereof according to any one of (1) to (4), in which the step (A) is a step of filtrating the aqueous solution including the terephthalic acid or the salt thereof and the diol through the polyamide-based nanofiltration membrane while maintaining and/or controlling a temperature at 30°C or higher and 45°C or lower.
(6) The method for producing terephthalic acid or a salt thereof according to any one of (1) to (5), in which a nanofiltration membrane treatment in the step (A) is a filtration treatment by a feed and bleed process.
(7) The method for producing terephthalic acid or a salt thereof according to any one of (1) to (6), in which the salt of terephthalic acid is disodium terephthalate.
(8) The method for producing terephthalic acid or a salt thereof according to any one of (1) to (7), the method further including a step (C) of acidifying the non-permeated liquid in the step (A) to crystallize the terephthalic acid.
(9) A method for producing a diol, the method including a step (D) of filtrating an aqueous solution including terephthalic acid or a salt thereof and a diol through a polyamide-based nanofiltration membrane to recover the diol from a permeated liquid.
(10) The method for producing a diol according to (9), in which the diol is ethylene glycol in the step (D).
(11) A method for producing a polyester, the method including: a step of producing terephthalic acid or a salt thereof by the method according to any one of (1) to (8); and a step of polymerizing a polyester using the terephthalic acid or the salt thereof obtained in the step as a raw material.
(12) A method for producing a polyester, the method including: a step of producing a diol by the method according to (9) or (10); and a step of polymerizing a polyester using the diol obtained in the step as a raw material.
(13) An apparatus for separating terephthalic acid or a salt thereof and a diol from an aqueous solution including the terephthalic acid or the salt thereof and the diol, the apparatus including: a holding tank configured to hold the aqueous solution; a polyamide-based nanofiltration membrane module; a filtrate recovery tank configured to recover a filtrate of the module; a non-permeated liquid recovery tank configured to recover a non-permeated liquid of the module; a flowmeter configured to measure a non-permeated liquid flow rate; and a control valve configured to be controlled by the flowmeter.
(14) The apparatus according to (13), in which the polyamide-based nanofiltration membrane has a molecular weight cutoff of 150 to 500.
(15) The apparatus according to (13) or (14), in which the holding tank includes a temperature sensor configured to detect a liquid temperature of a holding liquid in the holding tank and has a temperature adjustment function for the holding liquid in the holding tank controlled by the temperature sensor.
(16) The apparatus according to any one of (13) to (15), in which the holding tank includes a pH sensor configured to detect a pH of the holding liquid in the holding tank, and is connected to a pH adjustment mechanism for the holding liquid in the holding tank controlled by the pH sensor.
(17) A nanofiltration membrane for separating terephthalic acid or a salt thereof and a diol, including a functional layer including polyamide, and having a molecular weight cutoff of 150 to 500.

### ADVANTAGEOUS EFFECTS OF INVENTION

According to the present invention, terephthalic acid or the salt thereof and the diol can be selectively separated and recovered, and terephthalic acid or the salt, or the diol, which is selectively separated and recovered, can be independently reused as a polymer raw material such as polyester.

### BRIEF DESCRIPTION OF DRAWINGS

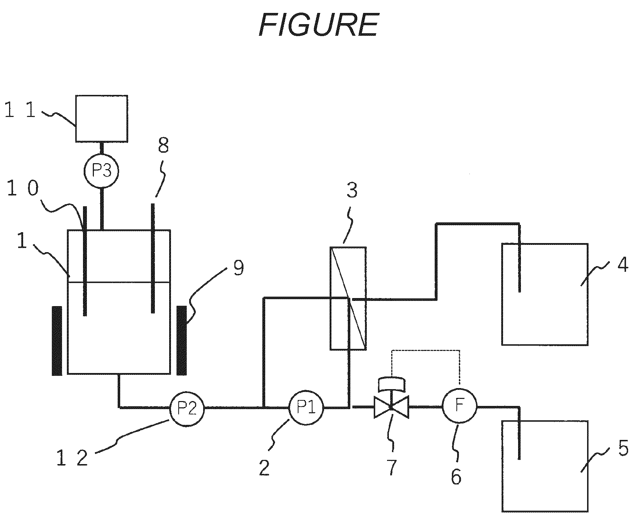

The FIGURE is a schematic view showing an aspect of a separation apparatus of the present invention.

### DESCRIPTION OF EMBODIMENTS

The present invention will be described in detail below, but the present invention is not limited thereto.

The present invention is a method for producing terephthalic acid or a salt thereof, in which an aqueous solution containing the terephthalic acid or the salt thereof and a diol is filtrated through a polyamide-based nanofiltration membrane to recover a solution of the terephthalic acid or the salt thereof as a non-permeated liquid (step (A)). By incorporating step (A) into a chemical recycle step of the terephthalic acid-based polyester, the terephthalic acid or the salt thereof derived from the terephthalic acid-based polyester can be recovered.

The terephthalic acid-based polyester is a polyester containing the terephthalic acid and the diol as monomer units, and specific examples thereof include polyethylene terephthalate (PET), isophthalate copolymer PET (PET/I), 1,4-cyclohexanedimethanol copolymer PET (PETG), spiroglycol copolymer PET, polytrimethylene terephthalate (PTT), and butylene terephthalate (PBT). A terephthalic acid-based polyester resin may be a mixture of two or more types. The terephthalic acid-based polyester is preferably the polyethylene terephthalate (PET).

The aqueous solution containing the terephthalic acid or the salt thereof and the diol can be prepared as a hydrolysate obtained by hydrolyzing the terephthalic acid-based polyester (step (B)). The hydrolysis of the terephthalic acid-based polyester refers to depolymerizing the terephthalic acid-based polyester to the terephthalic acid and the diol by hydrolyzing ester bonds of the terephthalic acid-based polyester in the presence of acids, alkalis, organic solvents, enzymes, metal catalysts, and the like. Preferably, alkaline hydrolysis in which the hydrolysis of the terephthalic acid-based polyester efficiently proceeds and a monomer is obtained in a state of the terephthalic acid or the salt thereof or enzymatic hydrolysis are preferred. In the alkaline hydrolysis, the hydrolysis proceeds by heating in a temperature range of 50°C to 250°C in the presence of caustic soda or ammonia. In addition to water, the hydrolysis may be performed in the presence of a polar solvent such as ethanol. The hydrolysis using an enzyme may also be performed. As the enzyme, a PET degrading enzyme (PET hydrolase) is already known, and an enzyme having high activity can be selected and used. The PET degrading enzyme is classified and defined with an enzyme number (EC number) of 3.1.1.101.

Specific examples of the terephthalate contained in the aqueous solution containing the terephthalic acid or a salt thereof and the diol include disodium terephthalate, dilithium terephthalate, and dipotassium terephthalate, and disodium terephthalate is preferred.

Specific examples of the diol contained in the aqueous solution containing the terephthalic acid or a salt thereof and the diol include ethylene glycol that can form polyester together with the terephthalic acid, 1,3-propanediol, and 1,4-butanediol, and the ethylene glycol is preferred.

A concentration of the aqueous solution containing the terephthalic acid or the salt thereof and the diol is not limited. The concentration is preferably 1 g/L to less than 200 g/L, and is more preferably in a range of 10 g/L to 100 g/L.

In the present invention, the polyamide-based nanofiltration membrane is used as a nanofiltration membrane.

The nanofiltration membrane is also called a nanofilter (nanofiltration membrane, NF membrane), and is a membrane generally defined as a "membrane that allows monovalent ions to pass through and blocks divalent ions". The nanofiltration membrane has minute gaps of about several nanometers, and is mainly used to block microparticles, molecules, ions, salts, and the like in water.

The polyamide-based nanofiltration membrane used in the present invention is a nanofiltration membrane in which a functional layer of the nanofiltration membrane includes polyamide, and examples of polyamide include linear polymers or crosslinked polymers containing aliphatic and/or wholly aromatic diamines as monomers. In addition, the polyamide-based nanofiltration membrane is not limited to a membrane formed of one type of material, and may be a membrane containing a plurality of membrane materials. Examples of a nanofiltration membrane other than the polyamide-based nanofiltration membrane include a polysulfone-based nanofiltration membrane whose functional layer is a polysulfone and a cellulose acetate-based nanofiltration membrane whose functional layer is a cellulose acetate, which are not preferable because the separation of terephthalic acid or the salt thereof and the diol is insufficient.

The nanofiltration membrane is generally defined as a "membrane that allows monovalent ions to pass through and blocks divalent ions". Although there is no correlation between technical characteristics thereof and a pore size of the membrane, an index of the molecular weight cutoff may be used to classify the pore size of the nanofiltration membrane. The molecular weight cutoff is well known to those skilled in the art as an index representing membrane performances of a separation membrane as "data obtained by plotting the molecular weight of the solute as the horizontal axis and the blocking rate as the vertical axis is called a molecular weight cutoff curve. The molecular weight at which the blocking rate is 90% is called the molecular weight cutoff of the membrane." described in Membrane Science Experiment Series, Volume III, Artificial Membrane Edition, edited by the Membrane Society of Japan, Editorial Committee/Naofumi Kimura Shinichi Nakao·Haruhiko Oya·Tsutomu Nakagawa (1993, Kyoritsu Publishing), P92. The blocking rate is an index representing a separation property of a substance to be separated by the separation membrane, which is calculated by Equation (1). blocking rate (%) of the target substance = (1 - concentration of the target substance on the membrane permeated side/concentration of the target substance on the membrane non- permeated side) × 100

The polyamide-based nanofiltration membrane used in the present invention preferably has a molecular weight cutoff in a range of 150 to 500. When the molecular weight cutoff of the polyamide-based nanofiltration membrane is less than 150, the diol is likely to be separated on the non-permeated liquid side together with terephthalic acid or the salt thereof, and an selective separation efficiency may decrease. On the other hand, when the molecular weight cutoff is more than 500, the selective separation efficiency of terephthalic acid or the salt thereof on the non-permeated liquid side of the nanofiltration membrane is maintained high, but a nanofiltration membrane permeability of terephthalic acid or the salt thereof may become high.

The separation efficiencies of terephthalic acid or the salt thereof and the diol can be increased by filtrating a liquid to be treated by the nanofiltration membrane while maintaining and/or controlling a Ph preferably at 5.7 or more and less than 11, more preferably 7.0 or more and less than 11, and further preferably 7.0 or more and 8.0 or less. When the pH is less than 5.7, the water solubility of terephthalic acid decreases, which causes membrane clogging. The polyamide-based nanofiltration membrane is characterized in that terephthalic acid or the salt thereof are selectively blocked whether terephthalic acid or the salt thereof is in a state of being dissociated into ions or dissolved in a non-dissociated state, but when the pH is 7.0 or more, since a proportion of terephthalic acid or the salt thereof in the non-dissociated state increases compared to that when the pH is less than 7.0, the blocking rate of terephthalic acid or the salt thereof to the nanofiltration membrane having polyamide as a separation functional layer is further improved, which is preferable. On the other hand, when the pH is 11 or more, the alkaline hydrolysis of the polyamide functional layer on a surface of the nanofiltration membrane proceeds, and membrane deterioration is promoted, which is not preferable.

A treatment temperature of the nanofiltration membrane is not limited as long as the treatment temperature is a temperature at which the deterioration of the nanofiltration membrane does not occur. The treatment temperature is preferably lower than 50°C, preferably 25°C or higher and lower than 50°C, and is more preferably 30°C or higher and 45°C or lower.

Examples of the polyamide-based nanofiltration membrane that can be preferably used in the present invention include NF series and NF99HF series manufactured by Alfa Laval K. K., NF-90 series, NF-200 series, NF-245 series, and NF-270 series manufactured by Filmtech Co., Ltd., NTR-7250 series and NTR-729 series manufactured by Nitto Denko Corporation, UTC 60 series manufactured by Toray Industries, Inc., NE40 series, NE70 series, NE90 series, HRM series, ARM 50 series, ARM 70 series, and SRM series manufactured by Toray Advanced Materials Korea Inc., DK series, DL series, and HL series manufactured by SUEZ company, and NFS series, NFX series, NFW series manufactured by Synder company, and SR3D series manufactured by KOCH company.

The blocking rate of terephthalic acid or the salt thereof by the nanofiltration membrane is preferably 40% or more, more preferably 50% or more, further preferably 70% or more, still more preferably 90% or more, and particularly preferably 95% or more.

The blocking rate of the diol by the nanofiltration membrane is preferably 20% or less, more preferably 15% or less, further preferably 10% or less, and particularly preferably 5% or less.

A nanofiltration membrane treatment method is not particularly limited and may be performed by a method known to those skilled in the art. Specifically, a batch process, a continuous process, a feed and bleed process, and the like are known, and the feed and bleed process is preferable. The feed and bleed process is a mode in which the filtration is continuously performed with the circulation of the non-permeated liquid, and any amount of a raw solution untreated with the nanofiltration membrane (hereinafter also simply referred to as a raw solution) is mixed with the circulating non-permeated liquid (hereinafter also referred to as a circulating permeation liquid) to be provided to the nanofiltration membrane. Alternatively, a part of the non-permeated liquid may be recovered from outside the system as a recovery non-permeated liquid without being circulated, and terephthalic acid or the salt thereof may be recovered from the recovery non-permeated liquid.

In a nanofiltration membrane treatment, terephthalic acid or the salt thereof is concentrated on the non-permeated liquid side, but when the concentration of terephthalic acid or the salt thereof in the aqueous solution supplied to the nanofiltration membrane is excessively high, a crystal component is generated in a flow channel, leading to clogging of the membrane and the flow channel, and therefore it is desirable to control the concentration to be not larger than a concentration at which the crystal component is not generated.

A method for adjusting the concentration of terephthalic acid or the salt thereof contained in the aqueous solution supplied to the nanofiltration membrane to a concentration range in which the crystal is not generated is not particularly limited, and for example, the generation of crystal can be prevented by setting the concentration of terephthalic acid or the salt thereof in the aqueous solution supplied to the nanofiltration membrane to less than 100 g/L. For example, when the non-permeated liquid is not circulated, a concentration of the raw solution may be set to less than 100 g/L, or may be set to a concentration equal to or less than a concentration at which the crystal component is not generated by diluting the non-permeated liquid of the nanofiltration membrane. When the nanofiltration membrane treatment is a feed and bleed process, a permeated liquid of the nanofiltration membrane and the same amount of raw solution as the recovery non-permeated liquid discharged outside the system are supplied. Since the circulating non-permeated liquid can be diluted by the supplied raw solution, the concentration of terephthalic acid or the salt thereof contained in the aqueous solution supplied to the nanofiltration membrane can be controlled to be below a certain level by controlling an amount of terephthalic acid or the salt thereof concentrated by the nanofiltration membrane treatment and an amount diluted by the supply of the raw solution by a flow amount of the recovery non-permeated liquid.

A method for recovering terephthalic acid or the salt thereof from the aqueous solution containing terephthalic acid or the salt thereof recovered as the nanofiltration membrane non-permeated liquid is not particularly limited, and may be any method known to those skilled in the art, and it is preferable to crystallize terephthalic acid by adding an acid to the nanofiltration membrane non-permeated liquid to acidify (step (C)). The crystallized terephthalic acid can be easily separated by centrifugal separation or membrane separation. According to this operation, the purity of terephthalic acid recovered on a non-permeated side portion of the nanofiltration membrane can be further improved.

Meanwhile, since a nanofiltration membrane permeated liquid contains the diol, the diol can be recovered from the non-permeated liquid (step (D)). A method for recovering the diol from the nanofiltration membrane permeated liquid is not particularly limited and a method known to those skilled in the art may be used, and energy can be saved by concentrating the diol using a reverse osmosis membrane.

Excess water can be recovered as a permeated liquid obtained by filtrating the nanofiltration membrane permeated liquid through the reverse osmosis membrane. The recovered excess water can be reused, such as by being used as a solvent in a step of hydrolyzing the terephthalic acid-based polyester to produce a solution containing terephthalic acid or the salt thereof and the diol.

The reverse osmosis membrane is also called an RO membrane, and is a membrane generally defined as a "membrane having a desalination function including monovalent ions". The reverse osmosis membrane is a membrane that is thought to have ultra-minute gaps of about several angstroms to several nanometers, and is mainly used for removing ionic components in seawater desalination and ultrapure water production.

Examples of the reverse osmosis membrane that can be preferably used in the present invention include a super low pressure type TMG series, TMHA series, TMG (D) series, TMG (P) series, SUL-G series, SUL-GTS series, TSW-LE series, TLF series, low pressure type SU-700 series, TM700 series, TM700D series, SU-700T series, TM800V series, TML (D) series, standard type TM 800M series, and high removal type TM 800K series which are polyamide-based reverse osmosis membranes modules and manufactured Toray Industries, Inc., cellulose acetate-based reverse osmosis membranes SC-L100R, SC-L200R, SC-1100, SC-1200, SC-2100, SC-2200, SC-3100, SC-3200, SC-8100, and SC-8200 manufactured by the same company, NTR-759HR, NTR-729HF, NTR-70SWC, ES10-D, ES20-D, ES20-U, ES15-D, ES15-U, and LF10-D manufactured by Nitto Denko Corporation, RO98pHt series, RO99 series, and RO90 series manufactured by manufactured by Alfa Laval K. K., SE series manufactured by SUEZ company, BW30-4040, TW30-4040, XLE-4040, LP-4040, LE-4040, SW30-4040, and SW30HRLE-4040 manufactured by Filmtec, RO series and HRX series manufactured by KOCH company, and ACM-1, ACM-2, and ACM-4 manufactured by TRISEP company.

A diol aqueous solution condensed on the non-permeated liquid side of the reverse osmosis membrane can be further evaporated and concentrated. The evaporative concentration is a method of increasing the concentration of the diol by heating and/or reducing the pressure of the diol aqueous solution to gasify and remove moisture. Examples of common device include evaporators, flash evaporators, multi-effect cans, spray drying, freeze drying, and the like.

In addition, as a pre-step of step (A) (post-step of step (B)), it is preferable to perform solid-liquid separation and/or an ultrafiltration membrane treatment on the liquid to be treated by the nanofiltration membrane.

A method of the solid-liquid separation is not particularly limited, but centrifugal separation using a screw decanter and membrane separation using a filter press, or precision filtration membrane (microfiltration) are preferable, and the membrane separation is more preferable.

A method of the ultrafiltration membrane treatment is not particularly particularly limited, as an ultrafiltration membrane to be used, an ultrafiltration membrane having a molecular weight cutoff of 500 Da to 200,000 Da, preferably 10,000 Da to 50,000 Da can be used. As a material of the ultrafiltration membrane, a membrane formed of a material such as polyethersulfone (PES), polyvinylidene difluoride (PVDF), or regenerated cellulose can be used. As a shape of the ultrafiltration membrane, a tubular type, a spiral element, a hollow fiber membrane, a flat membrane, or the like can be preferably used. Filtration by the ultrafiltration membrane includes a cross-flow method and a dead-end filtration method, and the cross-flow filtration method is preferable in terms of membrane fouling and flux.

Terephthalic acid or the salt thereof and the diol obtained by the present invention can be used as a raw material of the polyester. A method for producing the polyester is not particularly limited, and may be performed with a method known to those skilled in the art. For example, when a polyester to be produced is a polyethylene terephthalate, a method of esterifying terephthalic acid and ethylene glycol and producing a polyethylene terephthalate by a melt polymerization reaction is known. Further, the obtained polyethylene terephthalate can be made into a higher molecular weight polymer by solid-phase polymerization. At this time, only terephthalic acid or the salt thereof and the diol obtained in the present invention may be used as the raw material, or terephthalic acid or a salt thereof and a diol obtained by a method other than the present invention may be added. To modify the polyester, other compounds may be copolymerized or mixed.

As one aspect of an apparatus that separates terephthalic acid or the salt thereof and the diol from the aqueous solution containing terephthalic acid or the salt thereof and the diol, the FIGURE shows an aspect of an apparatus whose technical feature is to perform nanofiltration treatment by the feed and bleed process.

The apparatus in the FIGURE includes a holding tank (also referred to as a raw water tank) 1 for holding the aqueous solution containing terephthalic acid and the diol, a pump 2 for circulating the aqueous solution in the holding tank to a nanofiltration membrane module, a polyamide-based nanofiltration membrane module 3 for separating terephthalic acid or the salt thereof from the diol, a filtrate recovery tank 4 for recovering a filtrate containing the diol, a non-permeated liquid recovery tank 5 for recovering the non-permeated liquid discharged for recovering terephthalic acid or the salt thereof, a flowmeter 6 that measures a flow rate of the non-permeated liquid flowing into the non-permeated liquid recovery tank, and a control valve 7 controlled by the flowmeter.

The holding tank of the present apparatus may include a temperature sensor 8 that detects a temperature of the aqueous solution in the holding tank and a function 9 for adjusting the temperature of the aqueous solution in the holding tank controlled by the temperature sensor. Nanofiltration can be performed at a temperature suitable for separation between terephthalic acid or the salt thereof and the diol.

The holding tank of the present apparatus may be connected to a pH sensor 10 that detects a pH of the aqueous solution in the holding tank and a device 11 that supplies an alkali to the aqueous solution in the holding tank controlled by the pH sensor. Accordingly, the nanofiltration can be performed at a pH suitable for the separation between terephthalic acid or the salt thereof and the diol.

A pressure pump 12 may be connected between the holding tank and the circulation pump of the present apparatus. At this time, the non-permeated liquid may be supplied between the pressure pump and the circulation pump and circulated again. According to the present aspect, filtration by the feed-and-bleed method is possible.

### Examples

The present invention will be specifically described below with reference to Examples. However, the present invention is not limited thereto.

### (Reference Example 1) Preparation of Aqueous Solution for Membrane Separation Evaluation of Terephthalate and Diol

31.5 g of disodium terephthalate and 9.31 g of ethylene glycol were added to 3 L of RO water, followed by stirring at 25°C to dissolve them. A pH was adjusted to 5.7, 7.0, or 8.0 using 1 mol/L hydrochloric acid aqueous solution or 1 mol/L sodium hydroxide aqueous solution to obtain a terephthalate and ethylene glycol-containing aqueous solution for membrane separation evaluation.

### (Reference Example 2) Preparation of Aqueous Solution for Membrane Concentration of Diol

3.28 g/L of disodium terephthalate and 94.64 g of ethylene glycol were added to 3.9 L of RO water, followed by stirring at 25°C to dissolve them. The pH was adjusted to 8.0 using 1 mol/L sodium hydroxide aqueous solution to prepare an aqueous solution for membrane concentration of ethylene glycol.

### (Reference Example 3) Method for Measuring Terephthalic Acid Concentration

The terephthalic acid concentration was analyzed by an HPLC under the following conditions and was quantified as a concentration of non-dissociated terephthalic acid by comparison with a standard sample.
Column: Synerg HidroRP 4.6 mm × 250 mm (manufactured by Phenomenex)
Mobile phase: acetonitrile - 0.1% H₃PO₄ (flow rate 1.0 mL/min)
Detection method: UV (240 nm)
Temperature: 40°C

### (Reference Example 4) Method for Measuring Ethylene Glycol

The concentration of ethylene glycol was analyzed by an HPLC under the following conditions and was quantified by comparison with a standard sample.
Column: Shodex Sugar SH1011 (manufactured by Showa Denko K.K.)
Mobile phase: 0.05 M sulfuric acid aqueous solution, 0.6 mL/min
Detection method: RI
Temperature: 65°C

### (Comparative Example 1) Membrane Separation between Terephthalic Acid and Ethylene Glycol Using Polysulfone-Based Nanofiltration Membrane

Using 3 L of terephthalate and ethylene glycol-containing aqueous solution having a pH of 7.0 prepared in Reference Example 1, a separation property of terephthalic acid and ethylene glycol by the polysulfone-based nanofiltration membrane was evaluated. As a membrane separation device, "SEPA" (registered trademark) CF-II (effective membrane area 140 cm², GE W&PT) was used, and MPF-34 (molecular weight cutoff 200, Koch Separation Solutions) was used as the polysulfone-based nanofiltration membrane. 3 L of terephthalate and ethylene glycol-containing aqueous solution having a pH of 7.0 was put into the raw water tank, and a filtration treatment was performed at an operation temperature of 30°C, a membrane surface linear velocity of 20 cm/sec, and a permeation flow rate of 0.5 m/day. In a state in which all of the permeated liquid and non-permeated liquid returned to the raw water tank, the filtration was continued for 10 minutes for stabilization, and then 1 mL of each of the raw water tank and the permeated liquid was sampled. Table 1 shows results obtained by calculating the terephthalic acid concentration and the ethylene glycol concentration using the methods in Reference Examples 3 and 4 and blocking rates of terephthalic acid and ethylene glycol according to Equation (1).

### (Example 1) Membrane Separation of Terephthalic Acid and Ethylene Glycol Using Polyamide-Based Nanofiltration Membrane

A separation property of terephthalic acid and ethylene glycol was evaluated in the same manner as in Comparative Example 1 except that DK (molecular weight cutoff 150 to 300, SUEZ), DL (molecular weight cutoff 150 to 300), SR3D (molecular weight cutoff 200, Koch Separation Solutions), NFX (molecular weight cutoff 150 to 300, Synder), NFW (molecular weight cutoff 300 to 500, Synder), and NFG (molecular weight cutoff 600 to 800, Synder) were used as the polyamide-based nanofiltration membrane. The results are shown in Table 1.

From the results of Comparative Example 1 and Example 1, it was found that a blocking rate difference between terephthalic acid and ethylene glycol was larger and the selective separation efficiency calculated as "terephthalic acid blocking rate/ethylene glycol blocking rate" was higher in the case where a polyamide-based nanofiltration membrane was used than the case where a polysulfone-based nanofiltration membrane was used.

**Table 1**

| | Nanofiltration membrane | Membrane material | Molecular weight cutoff |
|---|---|---|---|
| Comp. Ex. 1 | MPF-34 | Polysulfone | 200 |
| Ex. 1 | DK | Polyamide | 150 to 300 |
| | DL | Polyamide | 150 to 300 |
| | SR3D | Polyamide | 200 |
| | NFX | Polyamide | 150 to 300 |
| | NFW | Polyamide | 300 to 500 |
| | NFG | Polyamide | 600 to 800 |

**Table 1 (continued)**

| | Raw water tank concentration (g/L) | | Permeated liquid concentration (g/L) | | Blocking rate (%) | | Selective separation efficiency |
|---|---|---|---|---|---|---|---|
| | Terephthalic acid | Ethylene glycol | Terephthalic acid | Ethylene glycol | Terephthalic acid | Ethylene glycol | |
| Comp. Ex. 1 | 7.96 | 2.96 | 0.07 | 2.25 | 99.1 | 24.0 | 4.13 |
| Ex. 1 | 8.55 | 3.05 | 0.70 | 2.99 | 91.8 | 2.0 | 45.9 |
| | 8.48 | 3.00 | 0.11 | 2.96 | 98.7 | 1.3 | 75.9 |
| | 8.00 | 3.02 | 0.04 | 2.82 | 99.5 | 6.6 | 15.1 |
| | 8.39 | 3.04 | 0.15 | 2.91 | 98.2 | 4.3 | 22.8 |
| | 8.44 | 2.99 | 0.22 | 2.92 | 97.4 | 2.3 | 42.3 |
| | 8.25 | 3.00 | 4.55 | 2.98 | 44.8 | 0.7 | 64.0 |

### (Example 2) Membrane Separation of Terephthalic Acid and Ethylene Glycol at pH 5.7-8.0 Using Polyamide-Based Nanofiltration Membrane

The separation property of terephthalic acid and ethylene glycol was evaluated in the same manner as in Comparative Example 1 except that DL (molecular weight cutoff 150 to 300), SR3D (molecular weight cutoff 200, Koch Separation Solutions), and NFX (molecular weight cutoff 150 to 300, Synder) were used as the polyamide-based nanofiltration membrane, and terephthalic acid · ethylene glycol-containing aqueous solution having a pH of 5.7, 7.0, or 8.0 prepared by the method in Reference Example 1 was used as an aqueous solution for evaluation of membrane separation of terephthalic acid and ethylene glycol. The results are shown in Table 2.

From the results, it was found that by performing the membrane separation in a pH range of 7.0 or more using a polyamide-based nanofiltration membrane, compared to the case where the membrane separation is performed at a lower pH, as the blocking rate of terephthalic acid increases, the blocking rate difference between terephthalic acid and ethylene glycol also increases, and the selective separation efficiency of terephthalic acid and ethylene glycol is further improved.

**Table 2**

| | Nanofiltration membrane | pH | Raw water tank concentration (g/L) | | Permeated liquid concentration (g/L) | |
|---|---|---|---|---|---|---|
| | | | Terephthalic acid | Ethylene glycol | Terephthalic acid | Ethylene glycol |
| | DL | 5.7 | 8.44 | 2.99 | 0.62 | 2.94 |
| | | 7.0 (Ex. 1) | 8.48 | 3.00 | 0.11 | 2.96 |
| | | 8.0 | 8.4 | 3.00 | 0.09 | 2.96 |
| | SR3D | 5.7 | 7.96 | 3.01 | 0.21 | 2.86 |
| Ex. 2 | | 7.0 (Ex. 1) | 8.03 | 3.02 | 0.04 | 2.87 |
| | | 8.0 | 7.94 | 3.10 | 0.03 | 2.96 |
| | NFX | 5.7 | 8.49 | 3.05 | 0.41 | 2.89 |
| | | 7.0 (Ex. 1) | 8.39 | 3.04 | 0.16 | 2.91 |
| | | 8.0 | 8.37 | 3.04 | 0.15 | 2.92 |

**Table 2 (continued)**

| | Blocking rate (%) | | Selective separation efficiency |
|---|---|---|---|
| | Terephthalic acid | Ethylene glycol | |
| | 92.7 | 1.7 | 54.5 |
| | 98.7 | 1.3 | 75.9 |
| | 98.9 | 1.3 | 76.1 |
| | 97.4 | 5.0 | 19.5 |
| Ex. 2 | 99.5 | 5.0 | 19.9 |
| | 99.6 | 4.5 | 22.1 |
| | 95.2 | 5.2 | 18.3 |
| | 98.1 | 4.3 | 22.8 |
| | 98.2 | 3.9 | 25.2 |

### (Example 3) Concentration of Ethylene Glycol Using Reverse Osmosis Membrane

Using 4 L of the aqueous solution for membrane concentration of ethylene glycol having a pH of 8.0 prepared in Reference Example 2, ethylene glycol was concentrated by the reverse osmosis membrane. As a membrane separation device, "SEPA" (registered trademark) CF-II (effective membrane area 140 cm², GE W&PT) was used, and a TM800V series flat membrane (salt removal rate 99.8%, Toray) was used as the reverse osmosis membrane. 4 L of aqueous solution for membrane concentration of ethylene glycol having a pH of 8.0 was put into the raw water tank, and a filtration treatment was performed at an operation temperature of 30°C, a membrane surface linear velocity of 20 cm/sec, and a permeation flow rate of 0.7 m/day. In a state in which all of the permeated liquid and non-permeated liquid returned to the raw water tank, the filtration was continued for 10 minutes for stabilization, only the permeated liquid was started to be removed outside the system, and the filtration was continued until a permeated liquid amount reached 3.3 L in total. 1 mL of each of the raw solution before the filtration was started, and the non-permeated liquid and the permeated liquid after the filtration was completed was sampled. The results obtained by measuring the terephthalic acid and ethylene glycol concentrations by the methods in Reference Examples 3 and 4 are shown in Table 3.

From these results, it became clear that the concentration of ethylene glycol can be increased by performing filtration with the reverse osmosis membrane as a previous step for recovering ethylene glycol by a distillation operation or the like by the present operation, and the cost and energy required for purification and recovery can be reduced.

**Table 3**

| | | Liquid amount (L) | Concentration (g/L) | | Ethylene glycol recovery rate (%) |
|---|---|---|---|---|---|
| | | | Terephthalic acid | Ethylene glycol | |
| Ex. 3 | Raw solution | 4.0 | 0.6 | 26.0 | - |
| | Concentrated liquid | 0.61 | 3.8 | 131 | 76.8 |

### (Example 4) Hydrolysis of Polyethylene Terephthalate (PET) by Enzyme and Membrane Separation of Terephthalic Acid and Ethylene Glycol Using Polyamide-Based Nanofiltration Membrane

### (1) Preparation of Expression Vector for PET Degrading Enzyme

The PET degrading enzyme was prepared based on Tournier Vet al., Nature, 580 (7802), 216-219 (2020). A nucleic acid sequence was designed in which a restriction enzyme SacI recognition site was added to 5' upstream of the PET degrading enzyme (PDB ID: 6THT) in the present document and a restriction enzyme PstI recognition site was added to 3' downstream of the PET degrading enzyme, and a nucleic acid of the PET degrading enzyme cloned into a pUC-GW vector was obtained by a gene synthesizing service of Genewiz. The obtained vector was digested by SacI and PstI (Takara Bio Inc.), a nucleic acid fragment of the PET degrading enzyme was separated and purified, followed by being introduced into SacI and PstI sites of pCold1 (manufactured by Takara Bio Inc.) using "Ligation-Convenience Kit" (manufactured by Nippon Gene Co., Ltd.).

### (2) Expression and Purification of PET Degrading Enzyme

An expression vector of the PET degrading enzyme obtained in (1) was introduced into E. coli BL21 (DE3) (manufactured by Nippon Gene Co., Ltd.) according to a protocol of the same company. An obtained transformant was cultured according to an expression protocol (https://catalog.takara-bio.co.jp/com/tech_info_detail.php?mode=2&masterid=M100003093&unitid=U100004327) of a pCold vector series, and the cultured cells were collected by centrifugal separation. Protein extraction and purification were performed according to a user manual of "TALON Metal Affinity Resin" (manufactured by Takara Bio Inc.). The purified protein was concentrated by a 10 kDa ultrafiltration membrane (manufactured by Sartorius AG Co., Ltd.), and finally, 10 mg/mL of PET degrading enzyme dissolved in a 50 mM sodium phosphate buffer (pH 8.0) was obtained.

### (3) Hydrolysis of PET by Enzyme

An amorphous PET film (GoodFellow : ES301445 order code: 177-292-64) was pulverized using a blender (Osaka Chemical Co., Ltd.: WB-1) under liquid nitrogen to prepare finely pulverized PET for enzymatic decomposition. A reaction solution, a stirrer, a substrate, and an enzyme were placed in a 1 L reagent bottle, and an enzyme reaction was performed using a hot stirrer (Tokyo Rikakikai Co., Ltd.: RCH-1000). The obtained enzyme reaction solution was centrifuged at 5000 rpm for 10 minutes, and a supernatant thereof was used as a PET hydrolysate. The operation was repeated to prepare a total of 2 L of enzymatic PET hydrolysate. The results obtained by measuring the concentrations of terephthalic acid and ethylene glycol by the methods described in Reference Examples 3 and 4 are summarized in Table 4.

### [Enzymatic Reaction Conditions]

Reaction solution amount: 1000 (mL)
Hot stirrer set temperature: 70°C
Reaction solution composition: 1 M sodium phosphate buffer (pH 8.0)
Substrate amount: 3 (% by weight)
Stirring speed: 400 (rpm)
Reaction time: 16 hours

**Table 4**

| | Concentration (g/L) | | pH after reaction |
|---|---|---|---|
| | Terephthalic acid | Ethylene glycol | |
| Ex. 4 | 17.6 | 6.6 | 6.76 |

### (4) Membrane Separation of Terephthalic Acid and Ethylene Glycol of PET Hydrolysate Using Polyamide-Based Nanofiltration Membrane

Using 2 L of the PET hydrolysate obtained in (3), membrane separation of terephthalic acid and ethylene glycol was performed using a polyamide-based nanofiltration membrane. As membrane separation device, "SEPA" (registered trademark) CF-II (effective membrane area 140 cm², GE W&PT) was used, and DL (manufactured by SUEZ) was used as the polyamide-based nanofiltration membrane. 2 L of PET hydrolysate was put into the raw water tank, and a filtration treatment was performed at an operation temperature of 30°C, a membrane surface linear velocity of 20 cm/sec, and a permeation flow rate of 0.5 m/day. In a state in which all of the permeated liquid and non-permeated liquid returned to the raw water tank, the filtration was continued for 10 minutes for stabilization, only the permeated liquid was started to be removed outside the system, and the filtration was continued until a permeated liquid amount reached 1.5 L in total. 1 mL of each of the raw solution before the filtration was started, and the non-permeated liquid and the permeated liquid after the filtration was completed was sampled. The results obtained by measuring the terephthalic acid and ethylene glycol concentrations by the methods in Reference Examples 3 and 4 are shown in Table 5.

**Table 5**

| | | Liquid amount (L) | Concentration (g/L) | | Recovery rate (%) | |
|---|---|---|---|---|---|---|
| | | | Terephthalic acid | Ethylene glycol | Terephthalic acid | Ethylene glycol |
| Ex. 1 | Non-permeated liquid | 0.49 | 68.5 | 6.8 | 95.3 | 25.2 |
| | Permeated liquid | 1.5 | 0.63 | 6.5 | 2.7 | 74.2 |

### (Example 5) Recovery of Terephthalic Acid by Crystallization from Non-Permeated Liquid Obtained by Filtrating PET Hydrolysate through Polyamide-Based Nanofiltration Membrane

A 1N hydrochloric acid aqueous solution was added to 0.05 L of the non-permeated liquid obtained in Example 4, and the pH was adjusted to 3.0 to obtain a terephthalic acid crystallization aqueous solution. The entire amount of the solution whose pH was adjusted to 3.0 was filtrated using a suction filter (reusable filter unit manufactured by Advantech Inc.) and glass filter paper (GC-25 manufactured by Advantech Inc.). 10 mL of a 0.001 N hydrochloric acid aqueous solution was used to wash the terephthalic acid crystallization aqueous solution attached to the container, and crystals were washed. The crystals were dried to obtain 3.35 g (yield 97.8%) of terephthalic acid crystals.

### (Example 6) Recovery and Concentration of Ethylene Glycol by Reverse Osmosis Membrane from Permeated Liquid Obtained by Filtrating PET Hydrolysate through Polyamide-Based Nanofiltration Membrane

Using 1.5 L of the permeated liquid obtained in Example 4, ethylene glycol was concentrated by the reverse osmosis membrane. As a membrane separation device, "SEPA" (registered trademark) CF-II (effective membrane area 140 cm², GE W&PT) was used, and a TM800V series flat membrane (salt removal rate 99.8%, Toray) was used as the reverse osmosis membrane. 1.5 L of permeated liquid obtained in Example 4 was put into the raw water tank, and a filtration treatment was performed at an operation temperature of 30°C, a membrane surface linear velocity of 20 cm/sec, and a permeation flow rate of 0.7 m/day. In a state in which all of the permeated liquid and non-permeated liquid returned to the raw water tank, the filtration was continued for 10 minutes for stabilization, only the permeated liquid was started to be removed outside the system, and the filtration was continued until a permeated liquid amount reached 1.0 L in total. 1 mL of each of the raw solution before the filtration was started, and the non-permeated liquid and the permeated liquid after the filtration was completed was sampled. The results obtained by measuring the terephthalic acid and ethylene glycol concentrations by the methods in Reference Examples 3 and 4 are shown in Table 6.

**Table 6**

| | | Liquid amount (L) | Concentration (g/L) | | Recovery rate (%) | |
|---|---|---|---|---|---|---|
| | | | Terephthalic acid | Ethylene glycol | Terephthalic acid | Ethylene glycol |
| Ex. 6 | Non-permeated liquid | 0.5 | 1.8 | 17.5 | 99.5 | 89.7 |
| | Permeated liquid | 1.0 | 0.001 | 1.0 | 0.1 | 10.2 |

### (Example 7) Effect of Temperature on Separation Property of Terephthalic Acid or Salt Thereof and Diol by Polyamide-Based Nanofiltration Membrane

According to the procedure described in Reference Example 1, 3 L of the terephthalate and ethylene glycol-containing aqueous solution having a pH of 7.0 was prepared. Using the present aqueous solution, a operation temperature was changed to 25°C, 30°C (Example 1), 35°C, 40°C, and 45°C, and a membrane separation test was performed using the polyamide-based nanofiltration membrane and the SR3D. Other separation conditions and measurement methods are the same as those in Comparative Example 1. As shown in the results in Table 7, it was found that the selective separation efficiency is good in a range of 30°C to 45°C, and 35°C is particularly suitable.

**Table 7**

| Nanofiltration membrane | Temperature (°C) | Raw water tank concentration (g/L) | | Permeated liquid concentration (g/L) | |
|---|---|---|---|---|---|
| | | Terephthalic acid | Ethylene glycol | Terephthalic acid | Ethylene glycol |
| SR3D | 25 | 8.56 | 3.60 | 0.08 | 3.41 |
| | 30 (Ex. 1) | 8.03 | 3.02 | 0.04 | 2.87 |
| | 35 | 8.61 | 3.56 | 0.09 | 3.43 |
| | 40 | 8.56 | 3.60 | 0.10 | 3.42 |
| | 45 | 8.68 | 3.60 | 0.11 | 3.43 |

**Table 7 (continued)**

| Nanofiltration membrane | Temperature (°C) | Blocking rate (%) | | Selective separation efficiency |
|---|---|---|---|---|
| | | Terephthalic acid | Ethylene glycol | |
| SR3D | 25 | 99.1 | 5.28 | 18.8 |
| | 30 (Ex. 1) | 99.5 | 4.97 | 20.0 |
| | 35 | 98.9 | 3.84 | 25.7 |
| | 40 | 98.8 | 5.01 | 19.7 |
| | 45 | 98.7 | 4.69 | 21.0 |

### (Example 8) Effect of Concentration of Terephthalic Acid or Salt Thereof Contained in Aqueous Solution Supplied to Polyamide-Based Nanofiltration Membrane

The disodium terephthalate was dissolved in 3 L of RO water such that final concentrations were 50 g/L, 60 g/L, 70 g/L, 80 g/L, 90 g/L, and 100 g/L, and a terephthalate aqueous solution was prepared by adjusting the pH to 7.0 using the same procedure as described in Reference Example 1. The terephthalate aqueous solution was used as a raw solution to perform a filtration test using the polyamide-based nanofiltration membrane. The test method was performed in the same manner as in Comparative Example 1 except that the type of used membrane was SR3D. As a result of the filtration test, when the filtration treatment was performed on the raw solution of a disodium terephthalate to the concentration of 90 g/L, the filtration treatment could be continued at the blocking rate of 98% or more, and when the filtration treatment was performed on 100 g/L of the raw solution, precipitation was observed in the circulation flow channel of the non-permeated liquid due to excessive concentration of disodium terephthalate.

### INDUSTRIAL APPLICABILITY

Terephthalic acid or the salt thereof obtained in the present invention can be used as a raw material of terephthalic acid-based polyester.

### REFERENCE SIGNS LIST

1: holding tank
2: circulation pump
3: nanofiltration membrane module
4: filtrate recovery tank
5: non-permeated liquid recovery tank
6: flowmeter
7: control valve
8: temperature sensor
9: temperature adjustment function
10: pH sensor
11: alkali supply device
12: pressure pump

## Claims

1. A method for producing terephthalic acid or a salt thereof, the method comprising a step (A) of filtrating an aqueous solution comprising terephthalic acid or a salt thereof and a diol through a polyamide-based nanofiltration membrane to recover the terephthalic acid or the salt thereof from a non-permeated liquid.

2. The method for producing terephthalic acid or a salt thereof according to claim 1, the method further comprising a step (B) of hydrolyzing a terephthalic acid-based polyester to produce the aqueous solution comprising the terephthalic acid or the salt thereof and the diol.

3. The method for producing terephthalic acid or a salt thereof according to claim 1 or 2, wherein the polyamide-based nanofiltration membrane has a molecular weight cutoff of 150 to 500.

4. The method for producing terephthalic acid or a salt thereof according to any one of claims 1 to 3, wherein the step (A) is a step of filtrating the aqueous solution comprising the terephthalic acid or the salt thereof and the diol through the polyamide-based nanofiltration membrane while maintaining and/or controlling a pH at 7.0 or more and less than 11.

5. The method for producing terephthalic acid or a salt thereof according to any one of claims 1 to 4, wherein the step (A) is a step of filtrating the aqueous solution comprising the terephthalic acid or the salt thereof and the diol through the polyamide-based nanofiltration membrane while maintaining and/or controlling a temperature at 30°C or higher and 45°C or lower.

6. The method for producing terephthalic acid or a salt thereof according to any one of claims 1 to 5, wherein a nanofiltration membrane treatment in the step (A) is a filtration treatment by a feed and bleed process.

7. The method for producing terephthalic acid or a salt thereof according to any one of claims 1 to 6, wherein the salt of terephthalic acid is disodium terephthalate.

8. The method for producing terephthalic acid or a salt thereof according to any one of claims 1 to 7, the method further comprising a step (C) of acidifying the non-permeated liquid in the step (A) to crystallize the terephthalic acid.

9. A method for producing a diol, the method comprising a step (D) of filtrating an aqueous solution comprising terephthalic acid or a salt thereof and a diol through a polyamide-based nanofiltration membrane to recover the diol from a permeated liquid.

10. The method for producing a diol according to claim 9, wherein the diol is ethylene glycol in the step (D).

11. A method for producing a polyester, the method comprising: a step of producing terephthalic acid or a salt thereof by the method according to any one of claims 1 to 8; and a step of polymerizing a polyester using the terephthalic acid or the salt thereof obtained in the step as a raw material.

12. A method for producing a polyester, the method comprising: a step of producing a diol by the method according to claim 9 or 10; and a step of polymerizing a polyester using the diol obtained in the step as a raw material.

13. An apparatus for separating terephthalic acid or a salt thereof and a diol from an aqueous solution comprising the terephthalic acid or the salt thereof and the diol, the apparatus comprising: a holding tank configured to hold the aqueous solution; a polyamide-based nanofiltration membrane module; a filtrate recovery tank configured to recover a filtrate of the module; a non-permeated liquid recovery tank configured to recover a non-permeated liquid of the module; a flowmeter configured to measure a non-permeated liquid flow rate; and a control valve configured to be controlled by the flowmeter.

14. The apparatus according to claim 13, wherein the polyamide-based nanofiltration membrane has a molecular weight cutoff of 150 to 500.

15. The apparatus according to claim 13 or 14, wherein the holding tank comprises a temperature sensor configured to detect a liquid temperature of a holding liquid in the holding tank and has a temperature adjustment function for the holding liquid in the holding tank controlled by the temperature sensor.

16. The apparatus according to any one of claims 13 to 15, wherein the holding tank comprises a pH sensor configured to detect a pH of the holding liquid in the holding tank, and is connected to a pH adjustment mechanism for the holding liquid in the holding tank controlled by the pH sensor.

17. A nanofiltration membrane for separating terephthalic acid or a salt thereof and a diol, comprising a functional layer comprising polyamide, and having a molecular weight cutoff of 150 to 500.
